# EUROPEAN PATENT APPLICATION

(11) **EP 0 753 309 A2**
(43) Date of publication of application: **15.01.1997**
(21) Application number: 96830376.8
(22) Date of filing: 03.07.1996
(51) Int. Cl.: A61K 38/40

(54) **Preparation of lactoferrin (or analogous proteins) and desferrioxamine methanesulfonate (or other metal ion chelators) for the therapy of viral infectious diseases**

(30) Priority: 12.07.1995 IT RM950472
(71) Applicant: Gambit International Limited, Tortola (VG)
(72) Inventor: Valenti, Piera, 00153 Rome (IT); Antonini, Giovanni, 00153 Rome (IT)

(57) **Abstract**

The present invention relates to the therapeutic utilizzation of the preparation of lactoferrin and desferrioxamine metalsulphonate for the therapy of many acute or recurrent viral infectious diseases in humans and animals. In details, the present invention demonstrates the antiviral activity, based on the inhibition either of the absorption either of the replication of several virus, possessed by a preparation of lactoferrin (or its anologous proteins like transferrins) in apo or iron or other metal ions saturated forms, together with desferrioxamine metansulfonate (or other metal ion chelators like 8-idroxiquinoline 1,10-phenanthroline, phosphonoacetic acid). This antiviral activity is well evident towards DNA virus; lik*e Herpesviruses*, and towards RNA virus, like *Rhinovirus*, and can be generally extended and utilized for the therapy of many acute or recurrent viral infections concerning skin, mucosas or other tissues.

## Description

### TECHNICAL FIELD

The present invention relates to the therapeutic utization of a preparation of lactoferrin (or analogous proteins) in association with the iron chelator desferrioxamine methanesulfonate (or other metal ion chelators) for preventing and curing many viral infectious diseases in humans.

### BACKGROUND ART

It is well known that virus can multiply at expenses of energy processes of the host cells. It is also well known that the first step of a viral infectious disease involves the binding of the viral particles to specific structures in the membrane of the host cell. These early interactions are followed by the intracellular phase of the virus consisting in the viral integration in the cell genome or the viral replication causing the lysis of the cell and the spreading of the infection. The integation of the viral genome in the cell genome allows the virus to avoid the host immune defenses and does not cause the lysis of the host cell as long as the viral genome remains integrated without multiplying. In some circumstances, the integrated viral DNA can start a new replication cycle, causing the cell lysis and, therefore, recurrent infections.

The peculiar life of virus makes difficult to find substances with antiviral properties able to prevent the replication of these pathogen agents without affecting the host cell. Furthermore, the antiviral drugs generally reduce the moltiplication and the spreading of the virus in acute infections whereas are not able to avoid viral adsorption to host cells. For example, the infectious disease by *Rhinovirus* lack a suitable pharmacological therapy.

In order to inhibit the viral replication, an approach can be represented by hindering the early interactions virus-cell preventing the virus internalization or inibiting the inactivating synthesis of the viral enzymes necessary for the multiplication cycle.

Concerning the first therapeutic approach, i.e. the inhibition of the early interaction virus-cell, many substances have been testing in the last years but just some of them are suitable to act through specific binding sites either to cell receptor either to viral anti-receptor. Recently, it has been demonstrated an antiviral function of lactoferrin, a glycoprotein able to bind 2 atoms of Fe³⁺ per molecule.

The antiviral function of lactoferrin, that was indipendent to this iron chelating property, is related to a specific binding with the host surface. In fact, it has been demonstrated that lactoferrin (human and bovine) specifically binds to heparan-sulphate, a common proteoglycan which is a multifunctional constituent of mammalian cell membranes and of extracellular matrix. Heparan-sulfate is also involved in the receptorial site that plays a role in the early interactions of HSV1 and other virus. Thus lactoferrin is able to selectively inhibit the early interactions between virus and host cell and, consequently is able to prevent the internalization.

*In vivo* and in the common media for cell cultures, lactoferrin is partially saturated in its iron binding sites. In this condition, lactoferrin assumes a "close" shape that allows it to better bind the surface receptors of the host cells. This binding between iron saturated lactoferrin and the receptor inhibits the adsorption of virus to the host cell while allows Fe³⁺ ions to penetrate inside the cell. Successively, the release of chelated ferric ions could prevent a therapeutic use of lactoferrin as antiviral agent, possibly because the iron transportated into the cell (necessary for the viral replication) facilitates the moltiplication of the virus particles eventually present in the cells.

The inhibition of the viral replication, is an example of the second therapeutic approach. It has been widely demonstrated that the activity of the viral replication is correlated to the presence of metal ions such as Fe^{3+,} Zn²⁺ , Cu²⁺ and several molecules, known to be metal ion chelators, possess antiviral activity. In particular, chelators like 8-hydroxyquinoline, 1,10-phenanthroline, phosphonoacetic acid and desferrioxamine methanesulfonate are able to inhibit the viral moltiplication. From the above chelators, desferrioxamine metansulfonate, an hydroxamic siderophore produced by *Streptomyces pilosus*, utilized in the therapy of hemochromatosis, appears to be very active since it is able to chelate with high affinity intracellular Fe³⁺ ions having low affinity towards other metal ions.

Until now, the therapeutical antiviral utilization of these chelators was not made possible because these molecules, even if they are able to slow down the viral moltiplication but do not prevent the penetration of the virus into the cell.

*Herpesuvirdae* is a family of viruses that are responsible of acute and recurrent infectious diseases. In particular, *Herpes simplex* virus 1 (HSV1), is a virus that affects up to 90% of adults. The virus, after the first infection that is often clinically silent, can remain in latent form in skin and nervous cells. The virus is responsible of diseases such as lips or genital infection, primary gingival stomatitis, herpetic keratitis and umbilical skin infections. After events like exposure to sunlight, fever or stress, the virus can reactivate itself causing the appearing of painful and boring skin vesicles that hurt for several days even when the triggering cause is disappeared. Main locations of the recurrent infection are lips, genitals, face and sacrum.

At the moment, the treatments in use for the therapy of *Herpes simples* infections are preventive (sunlight filters on lips) or antiviral (chemotherapeutic drugs inhibitng the viral replication). However, the first one prevents just the viral reactivation after sunlight exposure and the latter is potentially toxic and just reduces the intensity and length of symptoms but is not able to prevent the recurrence of the infections.

*Rhinovirus* are casual agents of the common cold. For this virus it is not possible the formulation of an effective vaccine, because of the 115 antigenic varieties found. The relative harmlessness of the above viral infections does not suggest the therapeutic utilization of antiviral drugs that always possess toxicity towards the host.

### DISCLOSURE OF THE INVENTION

The present disclosure concerns the preparation of a mixture of at least one transferrin like lactoferrin (and, in a more general way, of proteins analogous to lactoferrin) and of at least one metal ion chelators such as desferrioxamine methanesulfonate or 8-hydroxyquinoline, 1,10-phenanthroline, phosphonoacetic acid, whose antiviral activity, that is here demonstrated to be exerted through the inhibition of adsorption and replication of several viruses, can be therapeutically utilized.

The therapeutic model, described in the present invention is based on the synergistic antiviral action of lactoferrin (or analogous proteins) and desferrioxamine metansulfonate (or other metal ion chelators). The antiviral activity of this association, not yet described, is much higher than that showed separately by the two components because their antiviral activity is exerted on two different phases of the viral cycle (adsorption and replication). It, therefore, makes obvious the therapeutic benefit of such an association towards viral infectious diseases in comparison with the common antiviral therapies that are focused on the inhibition of viral replication and show toxic effects towards the host organism.

Lactoferrin, which possesses very low or no toxic effect, and the chelator desferrioxamine methanesulfonate, already utilized in the human therapy, can be used at topic and systemic level against viral infections concerning skin and mucous membranes like nasal, oropharynx mucosa, intestinal, bronchial, vaginal and other mucosas.

As an example, we showed the antiviral activity towards DNA and RNA viruses, possessed by the preparation of lactoferrin (or its analogous proteins) and desferrioxamine metansulfonate (or other metal ion chelators). The antiviral activity is made clear by the protection of cell lysis due to viral replication of HSV1 (choosed as DNA virus) and *Rhinovirus* (choosed as RNA virus). The antiviral activity of the preparation of lactoferrin (or its analogous proteins) and desferrioxamine metansulfonate (or other metal ion chelators), is made evident by the absence or reduced presence of lysis plaques when the virus is added to cells cultured in the presence of this association. Since the antiviral activity of the preparation of between lactoferrin (or its analogous proteins) and desferrioxamine metansulfonate (or other metal ion chelators) is exerted towards either DNA viruses (like HSV1) either RNA viruses (like *Rhinovirus*,), is pointed out the possibility of an utilization of this association also for preventing and treating other acute or recurrent viral infections.

### WAYS OF CARRYING OUT THE INVENTION

A particularly preferred preparation according to the invention is a mixture of the two compounds mentioned above, and thus a mixture of lactoferrin at a concentration of 15 to 99% methansulfonate and desferoxamine at a concentration af 1 to 95% these concentration being given by weith respect to the mixture. By way of example, a preparation according to the invention can comprise 98% lactoferrin and 2% desferoxamine methansulfonate.

Lactoferrin from bovine milk is the particularly preferred protein according to the invention. It appears clear, from the example reported below, that bovine lactoferrin can be considered optimum, with respect to treatments currently in use for the prevention and cure of viral infectious diseases although can be substituted by recombinant human lactoferrin in the case of systemic treatments. Furthermore, the extremely low toxicity of said protein contained in the formula according to the present invention is well-known, since is a "natural" substance extracted from bovine milk and is very similar, in its amino acid composition and structural configuration, to the homologous protein that is normally present in humans. This is an additional advantage of bovine lactoferrin with respect to the chemicals currently used in infections treatments. The protein that is used according to the present invention can be extract or can be obtained by recombinant-DNA technology or also by chemical synthesis.

The term lactoferrin designates a glycoprotein present in colostrum and milk and in many biological secretions and in the leucocyte granules of mammalians. Lactoferrin possesses an isoelectric point of 7.8, a molecular weight of about 83,000 Da and two sugar chains. Each molecule of lactoferrin binds 2 atoms of Fe³⁺ in presence of sodium bicarbonate. The main function of this protein is to reduce the amount of free iron in biological liquids, inhibiting the bacterial growth (the bacteriostatic property of lactoferrin is already well known) and decreasing the risk of forming free radicals due to the presence of not chelated iron. Lactoferrin belongs to a family of glycoprotein generally called "Transferrin", charactherized in so that they possess two binding sites for Fe³⁺ per molecule and a high degree of sequence homology among them.

Lactoferrin can be industrially produced by purification from bovine milk. We mainly used bovine lactoferrin in the experiments described in the present disclosure but the same antiviral activity is possessed by all the marketable proteins known as lactoferrin such as human lactoferrin and lactoferrin from mouse, produced by extraction or by recombinant DNA technique, and, therefore, the antiviral activity of lactoferrin has to be extended, as described in the present disclosure, to all the lactoferrins from various natural or synthetic sources.

Furthermore, we tested different Fe³⁺, Cu²⁺, Mn, Ni, Zn saturation percentage of the above glycoproteins, from Apo form to totally metal saturated form. All lactoferrin preparations possessing different metal saturation have showed similar antiviral activity when these preparations are tested alone or in association with desferrioxamine methanesulfonate or other metal ion chelators.

The antiviral activity towards viruses possessed by the preparation of lactoferrin and desferroxamine methansulfonate (or other metal ion chelators), described in this disclosure, is also possessed, even with a lower antiviral activity, by other proteins chemically and enzimatically modified and belonging to the "Transferrin family" as lactoferrin substitutes.

The term desferrioxamine methanesulfonate designates a metabolite produced by *Streptomyces pilosus* able to chelate 1 Fe³⁺ per molecule also in the intracellular space. Under a chemical point of view, desferrioxamine methanesulfonate belongs to the trihydroxamic siderophore class that can bind with high affinity Fe³⁺ ions and with lower affinity other metal ions like Al³⁺, Cu²⁺, Zn²⁺ and Co²⁺.

The antiviral activity of the preparation of lactoferrin (or its analogous proteins) and desferrioxamine methanesulfonate (or other metal ion chelators) described in the present disclosure is showed at not toxic concentrations of lactoferrin and desferrioxamine methanesulfonate towards cultured cells of different sources.

The antiviral activity towards DNA and RNA virus possessed by the association of lactoferrin (or its analogous proteins) and desferrioxamine methanesulfonate (or other metal ion chelators) is the main claim of this disclosure while it is not relevant, as regard with the antiviral activity towards virus described in this disclosure, the source of the lactoferrin and desferrioxamine methanesulfonate.

The formula according to the present invention can be obtained and stored in liquid form, as solutions at a concentration of 0.1 to 10%, e.g. 1 to 5%, weigth/volume (g/ml) of the mixture in solvents acceptable for pharmaceutical use, in particular water or hydroalcholic solvents such as water-ethanol mixtures, or in solid form (lyophilized, dried, frozen) and in the other commonly known forms of storage: for example immobilized or adsorbed on an inert support commonly used in the pharmaceutical field.

The formula according to the invention, can thus be used in the liquid form, or in solid form, with the concentration specified above.

In its form ready-for use the preparation according to the invention, can also comprise further conventional compounds as well as carriers, fillers, flavouring agents, preseravatives, surfactants, colorants and other adjuvants selected from those conventionally used for the various liquid or solid form preparations.

The formulation, according to the invention, can comprise antibacterial compounds such as quaternary ammonium compounds with one long chain alkyl on the nitrogen atom, alkali metal pyrophosphates and orthophosphates, halogenated bisphenols and halogenated diphenyl ethers, sodium benzoate, sodium salicylate, etc.

The formulation, according to the invention, can further comprise diluents, e.g. giycerin, propylene glycol, dioxalanes, glycerol, glycofurol, dimethylacetamide, ethyl lactate, alcohols, glycols,sorbitols, USP oils, NF oils, lecitthin, lanolin, ethyl oleate, isopropyl myristate, benzyl benzoate, petrolatum, cocoa butter mixts and gelifying substances e.g.: carboxy vinyl polymers, sodium alginate, agar, gelatin, cellulose derivatives₁ pectin, xantan gum, polyvinyl pyrrolidone, glycerol, propylene glycol or polyethylene glycol.

The formulation, according to the invention, in either ointment, gel powder, solution should be used for purposes of prevention of recurrent viral diseases, preferably twice a day. For purpose of treatment the frequency of use can be increased to 3 to 4 times a day.

The preparation of lactoferrin (or its analogous proteins) and desferrioxamine methanesulfonate (or other metal ion chelators) has been tested for its antiviral activity towards HSV1, as prototype pathogen DNA virus, through the inhibition of very early phases of the adsorption and viral replication following an experimental model described below.

The preparation of lactoferrin (or its analogous proteins) and desferrioxamine methanesulfonate (or other metal ion chelators) has been also tested for its antiviral activity towards *Rhinovirus* as prototype pathogen RNA virus, using an experimental model described below.

The utilization of the association of lactoferrin (or its analogous proteins) and desferrioxamine methanesulfonate (or other metal ion chelators) in the therapy of infections by HSV1 and *Rhinovirus* are, therefore, two of the possible therapeutic applications of the antiviral activity of the preparation of lactoferrin (or its analogous proteins) and desferrioxamine methanesulfonate (or other metal ion chelators), described in this disclosure.

In fact, the antiviral properties of the preparation of lactoferrin (or its analogous proteins) and desferrioxamine methanesulfonate (or other metal ion chelators) can make this association also valid for the topic or systemic therapy of many acute and recurrent infectious diseases by DNA and RNA pathogen virus concerning humans and animals.

The here reported examples point out that the therapeutic antiviral activity towards different viruses possessed by the preparation of lactoferrin (or its analogous proteins) and desferrioxamine methanesulfonate (or other metal ion chelators) described in the present disclosure, can be utilized in acute or recurrent viral infections and can be considered optimal as regards the commonly used therapeutic treatments because its very low or no toxic effects.

The following examples have been carried out on *in vitro* models that are considered valid for testing substances with antiviral activity.

### EXAMPLES

### Cell cultures

The cultured cell utilized are different according with the tested virus. For testing HSV1, VERO cells have been utilized while for testing *Rhinovirus* we utilized HeLa cells. Both cell lines have been cultured as monolayers at 37°C in Eagle's MEM containing 1.2 g/l NaCO₃, 2 mM glutamine, 100 U/ml penicillin, 0.1 mg/ml streptomycin and 10% heat inactivated faetal calf serum in 5% CO₂ on 24- well tissue culture clusters (costar) by loading 0.5 ml of a suspension of 2x10⁵ HeLa cells/ml.

Unless otherwise indicated, lactoferrin and ovotransferrin used in the following examples are 30 % iron-saturated.

### Example 1

### Activity of lactoferrin and desferrioxamine methanesulfonate and related substances on the very early interactions between cell and viruses belonging to different genera.

The utilized procedure is the following:
a) incubation at 4°C for 1 h of the tested substances with cultured cells. After this period, the monolayer is washed and incubated at 37°C to allow viral infection;
b) incubation at 4°C for 1 h of the tested substances with the tested viruses. After this period, the monolayer is washed and incubated at 37°C to allow viral infection;
c) incubation at 4°C for 1 h of the tested substances with the virus and the cell monolayers. After this period, the monolayer is incubated at 37°C to allow viral infection.

The data concerning the activity of the lactoferrin (or other substances) are expressed as percentage of lysis plaques in comparison with the control in which the virus is incubated in its (or their) absence. The cultured cell utilized are different according with the tested virus. For testing HSV1, VERO cells have been utilized, while for testing *Rhinovirus* we utilized HeLa cells. Both cell lines have been cultured as monolayers at 37°C in Eagle's MEM containing 1.2 g/l NaCO₃, 2 mM glutamine, 100 U/ml penicillin, 0.1 mg/ml streptomycin and 10% heat inactivated faetal calf serum in 5% CO₂ on 24- well tissue culture clusters (costar) by loading 0.5 ml of a suspension of 2x10⁵ HeLa cells/ml.

Lactoferrin and glycosilate ovotransferrin have been used at the concentration of 1 mg/ml; desferrioxamine and 8-idrossiquinoline at the concentrations of 0.03 mg/ml.

**Table 1**

| **Effect of transferrins and chelators on the very early cell-virus interactions.** | | | |
|---|---|---|---|
| **Conditions** | | **Plaque forming units (%)** | |
| | | **HSV1** | **RHINOVIRUS** |
| Control | | 100 | 100 |
| Bovine lactoferrin | a | 10 | 20 |
| | b | 0 | 10 |
| | c | 0 | 5 |
| Ovotransferrin | a | 100 | 100 |
| | b | 100 | 100 |
| | c | 90 | 80 |
| Glycosilate ovotransferrin | a | 30 | 20 |
| | b | 10 | 10 |
| | c | 10 | 10 |
| Desferrioxamine methanesulfonate | a | 50 | 80 |
| | b | 100 | 100 |
| | c | 40 | 60 |
| 8-hydroxyquinoline | a | 60 | 70 |
| | b | 100 | 100 |
| | c | 50 | 70 |

In Table 1 are reported the data relative to bovine lactoferrin, in this experimental conditions, more active than human lactoferrin. On the contrary, ovotransferrin shows a poor antiviral activity except when it is chemically increased its glycosilation degree. Concerning the inhibition of the very early cell-virus interactions, desfemoxamine methanesulfonate, like 8-hydroxyquinoline and other described chelators, a does not possess the same activity.

### Example 2

### Activity of transferrins and chelators on the replication of viruses belonging to different genera.

These experiments have been performed adding the tested substances at 37 °C to the monolayer preincubated for 1 h at 4°C in the presence of the different viruses. The data are reported in table 2.

**Table 2**

| **Effect of transferrins and chelators on the replication of different virus.** | | |
|---|---|---|
| **Conditions** | **Plaque forming units (%)** | |
| | **HSV1** | **RHINOVIRUS** |
| Control | 100 | 100 |
| Bovine lactoferrin | 100 | 100 |
| Ovotransferrin | 100 | 100 |
| Glycosilate ovotransferrin | 100 | 100 |
| Desferrioxamine methanesulfonate | 80 | 90 |
| 8-Hydroxyquinoline | 75 | 80 |

From the reported data, is evident that lactoferrin and other transferrins do not affect the intracellular phase and the replication of the viruses.

A light effect is observed just for desferrioxamine methanesulfonate and 8-hydroxyquinoline for their well known mechanism of binding the intracellular iron necessary for the viral activity.

Other experiments have been carried out using the same concentration of bovine lactoferrin and ovotransferrin completely saturated with different metal ions. The data are reported in Table 3.

**Table 3**

| **Effect of lactoferrin saturated with different metal ions on the replication of different virus.** | | |
|---|---|---|
| **Conditions** | **Plaque forming units (%)** | |
| | **HSV1** | **RHINOVIRUS** |
| Control | 100 | 100 |
| Bovine lactoferrin in apo form | 30 | 30 |
| Bovine lactoferrin iron saturated | 0 | 0 |
| Bovine lactoferrin zinc saturated | 40 | 40 |
| Bovine lactoferrin cupper saturated | 10 | 20 |
| Bovine lactoferrin manganese saturated | 0 | 0 |
| Bovine lactoferrin cobalt saturated | 50 | 30 |
| Bovine lactoferrin nichel saturated | 30 | 40 |

From the data reported in Table 3 it can be notice that lactoferrin also in saturated forms shows a noticeable antiviral activity.

### Example 3

### Activity of the transferrins-chelators association.

The maximum of the antiviral activity can be obtained when the substances (lactoferrin or giycosilate ovotransferrin and desfenrioxamine methanesulfonate or 8-hydroxyquinoline) exert all together their action i.e. the inhibition of the virus-cell interaction by lactoferrin or glycosilate ovotransferrin and the inhibition of replication by desferrioxamine methanesulfonate or 8-hydroxyquinoline.

The experimental procedure is described below:
a) incubation at 4°C for 1 h of different associations with cultured cells. After this period, the monolayer is washed and incubated at 37°C to allow viral infection;
b)incubation at 4°C for 1 h of different associations with the tested viruses. After this period, the monolayer is washed and incubated at 37°C to allow viral infection;
c) incubation at 4°C for 1 h of the different associations with the virus and the cell monolayers. After this period, the monolayer is incubated without washing at 37°C to allow viral infection.

**Table 4**

| **Activity of the transferrins-chelators associations.** | | | |
|---|---|---|---|
| **Conditions** | | **Plaque forming units (%)** | |
| | | **HSV1** | **RHINOVIRUS** |
| Control | | 100 | 100 |
| Bovine lactoferrin +desferrioxamine methanesulfonate | a | 0 | 0 |
| | b | 0 | 0 |
| | c | 0 | 0 |
| Glycosilate ovotransferrin +desferrioxamine methanesulfonate | a | 0 | 0 |
| | b | 0 | 0 |
| | c | 0 | 0 |
| Lactoferrin + 8-idroxiquinoline | a | 0 | 0 |
| | b | 0 | 0 |
| | c | 0 | 0 |
| Glycosilate ovotransferrin + 8-hydroxyquinoline | a | 0 | 0 |
| | b | 0 | 0 |
| | c | 0 | 0 |

It is well evident that in all the experimental conditions the viral replication is totally inhibited. In fact, the transferrins-chelators association of is active either on the very early cell-virus interaction either on the intracellular replication of the viruses, so exerting the maximum of the antiviral effect. Similar result were also obtained with lactoferrin or ovotransferrin saturated with different metal ions such as Mn²⁺, Cu²⁺, Zn²⁺, Co^{2+,} Ni²⁺.

### Example 4 - Lyophilized powder

Composition per 5 g

### Active ingredients:

Lactoferrin, from bovine milk, SIGMA Chemical Co., cat L4765, 4,8 g
Desferroxamine methanesulfonate, Ciba Geigy, 0.2 g

### Use

Dissolve into distilled water and inject i.m. once a day.

### Example 5 - Ointment

Composition per 100 g

### Active ingredients:

Lactoferrin, from bovine milk, SIGMA Chemical Co., cat L4765, 10 g
Desferroxamine methanesulfonate, Ciba Geigy, 0.1 g

### Carriers, preservatives agents:

Paraffin oil 10 g
Vaseline 80 g

### Use

Apply on the skin twice a day

### Example 6 - Cream

Composition per 100 g

### Active ingredients:

Lactoferrin, from bovine milk, SIGMA Chemical Co., cat L4765, 10 g
Desferroxamine methanesulfonate, Ciba Geigy, 0.1 g

### Carriers, preservatives agents:

Paraffin oil 6 g
Chlorocresol 0.1 g
Vaseline 15 g
Ketostearil alcohol 7.2 g
Polyethylene glycol monocethyl ether 1,8 g
Distilled water up to 100 g

### Use

Apply on the skin twice a day.

### Example 7 - Gel

Composition per 100 g

### Active ingredients:

Lactoferrin, from bovine milk, SIGMA Chemical Co., cat L4765, 10 g
Desferroxamine methanesulfonate, Ciba Geigy, 0.1 g

### Carriers, preservatives agents:

| | |
|---|---|
| Methyl-p-hydroxybenzoate | 100 mg |
| Propyl-p-hydroxybenzoate | 20 mg |
| Xantan gum | 4 g |
| Lecithin | 3 g |
| Vitamin E | 100 mg |
| Distilled water up to | 100 g |

### Use

Apply on the skin twice a day.

### Example 8 - Spreading powder

Composition per 100 g

### Active ingredients:

Lactoferrin, from bovine milk, SIGMA Chemical Co., cat L4765, 10 g
Desferroxamine methanesulfonate, Ciba Geigy, 0.1 g

### Carriers, preservatives agents:

| | |
|---|---|
| Glycine | 4 g |
| Zin oxide | 3.4 g |
| Corn starch | 80 g |
| Sodium metabisulfite | 100 mg |
| Silica | 1 g |

### Use

Spread on the skin twice a day

### Example 9 - Nasal spray

Composition per 100 g

### Active ingredients:

Lactoferrin, from bovine milk, SIGMA Chemical Co., cat L4765, 10 g
Desferroxamine methanesulfonate, Ciba Geigy, 0.1 g

### Carriers, preservatives agents:

| | |
|---|---|
| Methyl-p-hydroxybenzoate | 100 mg |
| Propyl-p-hydroxybenzoate | 20 mg |
| Sodium phosphate monobasic | 200 mg |
| Sodium EDTA | 50 mg |
| Distilled water up to | 100 g |

### Use

Spray into the nostrils twice a day

### Example 10 - Nasal drops

Composition per 100 ml

### Active ingredients:

Lactoferrin, from bovine milk, SIGMA Chemical Co., cat L4765, 10 g
Desferroxamine methanesulfonate, Ciba Geigy, 0.1 g

### Carriers, preservatives agents:

| | |
|---|---|
| Methyl-p-hydroxybenzoate | 100 mg |
| Propyl-p-hydroxibenzoate | 20 mg |
| Sodium chloride | 0.8 g |
| Distilled water up to | 100 ml |

### Use

Drop 0.5 mls of solution per each nostril twice a day

## Claims

1. Preparation for preventing and curing acute and recurrent viral infetious diseases concerning skin, mucosas and other human or animal tissues characterized in that it comprising at least one transferrin and at least one molecule selected from the groups of chelators of iron and other metal ions.

2. Preparation according to claim 1, wherein the transferrin, in apo or iron or other metal ions saturated forms, at a protein concentration of 5 to 99% is represented by the bovine or human lactoferrin or by other natural or chemically modified transferrin characterized by high sequence homology with the protein known as lactoferrin, for example glycosilate ovotransferrin.

3. Preparation according to claim 1, wherein the chelator of iron and other metal ions at a concentration of 1 to 95% is represented by desferrioxamine methanesulfonate or by other molecules with similar metal ion binding capability, for example 8-hydroxyquinoline, 1,10-phenanthroline and phosphonoacetic acid.

4. Preparation according to claims 1 and 2, wherein said transferrin proteins are selected from the group consisting of said proteins of natural origin, comprising proteins of human, animal and plant origin, obtained by recombinant DNA technology and chemically synthetized.

5. Preparation according to claims 1 and 3 wherein said iron chelating molecules are selected from the group consisting of said molecules of natural origin and chemically sinthetized molecules.

6. Preparation formed by the association of lactoferrin or analogous protein and desferrioxamine methanesulfonate or other metal ion chelator according to claims 1,2 and 3, 4 and 5 wherein it is utilized for preventing and curing acute and recurrent viral infecious diseases concerning skin, mucosas and other tissues of humans an animals caused by DNA virus for examples, those belonging to the *Herpesvirus* family.

7. Preparation formed by the preparation of lactoferrin, or analogous protein, and desferrioxamine methanesulfonate, or other metal ion chelator, according to claim 1,2 3, 4 and 5, wherein it is utilized for preventing and curing acute and recurrent viral infecious diseases concerning skin, mucosas and other tissues of humans and animals caused by RNA viruses for examples, those belonging to the *Picornavirus* family like *Rhinovirus*.

8. Preparation formed by the preparation of lactoferrin or analogous protein, and desferrioxamine methanesulfonate or other metal ion chelator according to claims 1,2,3, 4,5,6 and 7 wherein it is utilized in liquid, solid or immobilized form for preventing and curing viral infectious diseases.

9. Preparation according to claims 1,2,3,4,5,6 and 7 wherein said mixture is in a form further comprising one or more adjuvants selected from carriers, preservants, diluents and gelling agents.

10. Methods of using a preparation according to claim 1 for preventing or treating viral infectious diseases of humans and animals, comprising topical or systemic administering said preparation at least once a day.
